# EUROPEAN PATENT APPLICATION

(11) **EP 0 919 210 A1**
(43) Date of publication of application: **02.06.1999**
(21) Application number: 98121221.0
(22) Date of filing: 06.11.1998
(51) Int. Cl.: A61F 9/007

(54) **Probe-style aspirator with cutter**

(30) Priority: 10.11.1997 JP 325241/97
(71) Applicant: Shimadzu Corporation, Kyoto-shi, Kyoto 604-8511 (JP); Koide, Ryohei, Tokyo 108-0074 (JP); Mikata, Osamu, Tokyo 142-0054 (JP)
(72) Inventor: Ihara, Masahiro Shimadzu Co., Sanjyo Factory of 1, Kyoto-shi, Kyoto 604-8511 (JP); Ohtani, Fumihiko Shimadzu Co., Sanjyo Factory of 1, Kyoto-shi, Kyoto 604-8511 (JP); Koide, Ryohei, Tokyo 108-0074 (JP); Mikata, Osamu, Tokyo 142-0053 (JP)
(74) Representative: WILHELMS, KILIAN & PARTNER Patentanwälte

(57) **Abstract**

A second probe (3) is inserted inside a hollow first probe (2) such that it can move relative to the first probe (2). A portion of an object (5) to be aspirated present outside the first probe (2) can be introduced into the first probe (2) via a suction inlet (4) formed in the first probe (2). The suction inlet (4) is opened and closed by relative movement of the second probe (3) with respect to the first probe (2). A laser cutter mechanism is provided, whereby the portion of the object (5) inside the first probe (2) is separated from the remainder thereof located outside the first probe (2) by means of laser beam.

## Description

### TECHNICAL FIELD

The present invention relates to a probe-style aspirator with cutter, which can be used, for example, in retinas and vitreous body surgery in the field of ophthalmology.

### DESCRIPTION OF RELATED ART

In the field of ophthalmology, for example, with progress in clinical treatment, research, investigative measurement devices, surgical devices, and the like, retinas and vitreous body surgery is carried out commonly. In order to remove and draw away a vitreous body in vitrectomy surgery, a probe-style aspirator fitted with a cutter, known as an Ocutome (registered trademark), is used. The tip portion of this Ocutome comprises a hollow first probe having a small diameter and a hollow second probe which is inserted inside this first probe, a suction inlet being formed in the circular wall of the first probe. A portion of a vitreous body introduced into the first probe via the suction inlet receives a shearing force by means of rotation of the second probe with respect to the first probe, or by a piston-type movement. Thereby, the portion of the vitreous body introduced into the first probe is separated from the remainder of the vitreous body, as if cut by a guillotine. The separated vitreous body inside the first probe is sucked away by the second probe.

Using a conventional structure of a probe-style aspirator with cutter, it is difficult to achieve a perfect cut of a soft material such as vitreous fibers. Therefore, vitreous cord or vitreous beads, or the like, are aspirated without being cut, and hence there is a risk that this material will block up the interior of the small-diameter probe. Consequently, the size of material sucked into the probe has been limited by reducing the size of the suction inlet. However, suction efficiency with respect to vitreous material has fallen. Furthermore, since the surgeon is required to pay attention with respect to blockages during use, the surgery time is lengthened and the burden on the patient is increased. Moreover, although the suction pressure is variable, if an excessively high pressure is applied when vitreous material in the vicinity of the retina is aspirated, there is a risk that retinal tearing occurs due to the retina itself being aspirated.

It is an object of the present invention to provide a probe-style aspirator with cutter whereby the aforementioned problems are resolved.

### SUMMARY OF THE INVENTION

The probe-style aspirator with cutter according to the present invention comprises a hollow first probe, and a second probe inserted into the first probe such that it is capable of moving relative to the first probe. A suction inlet is formed in the first probe, in order that a portion of an object to be aspirated present outside the first probe can be introduced into the first probe via the suction inlet.

A first characteristic of the present invention is that the suction inlet is opened and closed by relative movement of the second probe with respect to the first probe, and a laser cutter mechanism, which separates the portion of the object inside the first probe from the remainder of the object outside the first probe by means of laser beam when the suction inlet is in a closed state, is provided.

By means of this constitution, the portion of the object to be aspirated inside the first probe is cut or disintegrated in the vicinity of the suction inlet by the laser beam. Thereby, the portion of the object inside the first probe can be separated reliably from the remainder of the object located outside the first probe, and hence it can be aspirated reliably without producing blockages. Moreover, since the suction inlet is closed during the separation of the object, it is possible to prevent the impacts due to the separating operation and the laser beam from leaking outside the first probe. Therefore, safety can be improved in cases where the device according to the present invention is used as a medical instrument in retinas and vitreous body surgery, and the like.

A second characteristic of the present invention is that a shearing force can be applied to the portion of the object inside the first probe by relative movement of the second probe with respect to the first probe to cut it from the remainder of the object outside the first probe; and a mechanism is provided for disintegrating the portion of the object inside the first probe in the vicinity of the suction inlet, in order that a blockage inside the probes is not caused.

By means of this constitution, even if the portion of the object inside the first probe cannot be cut from the remainder of the object outside the first probe by the shearing force, it is disintegrated in the vicinity of the suction inlet. Thereby, it is possible to aspirate the portion of the object inside the first probe without causing blockages. In this constitution, it is preferable that the suction inlet is opened and closed by relative movement of the second probe with respect to the first probe; and the mechanism disintegrates the portion of the object inside the first probe by laser beam, when the suction inlet is in a closed state. Thereby, the disintegrating of the portion of the object inside the first probe is carried out reliably by the laser beam. Moreover, since the suction inlet is closed during the disintegrating, it is possible to prevent impacts due to the disintegrating operation, the disintegrated object, and the laser beam from leaking outside the first probe. Consequently, safety is improved in cases where the device according to the present invention is used as a medical instrument for retinas and vitreous body surgery, and the like.

The probe-style aspirator with cutter according to the present invention can be used as a medical instrument, for instance, as a vitrectomy instrument in the field of ophthalmology, thereby enabling surgery time to be shortened and the burden on the patient to be reduced.

The disintegrating of the object can be carried out simultaneously with the application of a shearing force, or it can be carried out prior to aspiration of the object into the second probe, provided that blocking of the probes is prevented. Irradiation of laser beam can be carried out continuously for an arbitrary period of time, or it can be carried out intermittently at an arbitrary time intervals. Desirably, the laser beam is guided by an optical fiber to the vicinity of the irradiation point.

In the present invention, it is preferable that an optical fiber for emitting laser beam is provided, and the laser beam emitting section of this optical fiber is prevented from contamination by a fluid conveyed thereto.

By means of the present invention, even a material which is difficult to cut, such as a vitreous body, can be aspirated reliably and continuously, without causing blockages. Also, by applying the present invention to a medical instrument for retinas and vitreous body surgery, and the like, it is possible to provide a probe-style aspirator with cutter whereby surgery time is shortened and the burden on the patient is reduced. Therefore, desirably, the probe-style aspirator with cutter according to the present invention is used as a medical instrument. In this way, treatment efficiency is improved, treatment time is shortened and the burden on the patient is reduced.

### BRIEF DESCRIPTION OF THE DRAWINGS

Fig. 1(1) is a vertical sectional view of a probe-style aspirator with cutter according to a first embodiment of the present invention; Fig. 1(2) is a horizontal sectional view of the principal part thereof.
Fig. 2(1) is a vertical sectional view of a probe-style aspirator with cutter according to a second embodiment of the present invention; Fig. 2(2) is a horizontal sectional view of the principal part thereof.
Fig. 3(1) is a vertical sectional view of an object to be aspirated prior to shearing in a probe-style aspirator with cutter according to a third embodiment of the present invention; Fig. 3(2) is a vertical sectional view of same with the suction inlet in a closed state; Fig. 3(3) is a vertical sectional view of same after disintegrate of the object to be aspirated.
Fig. 4(1) is a vertical sectional view of a probe-style aspirator with cutter according to a fourth embodiment of the present invention; Fig. 4(2) is a horizontal sectional view of the principal part thereof.

### DESCRIPTION OF THE PREFERED EMBODIMENTS

Below, embodiments of the present invention are described with reference to the drawings.

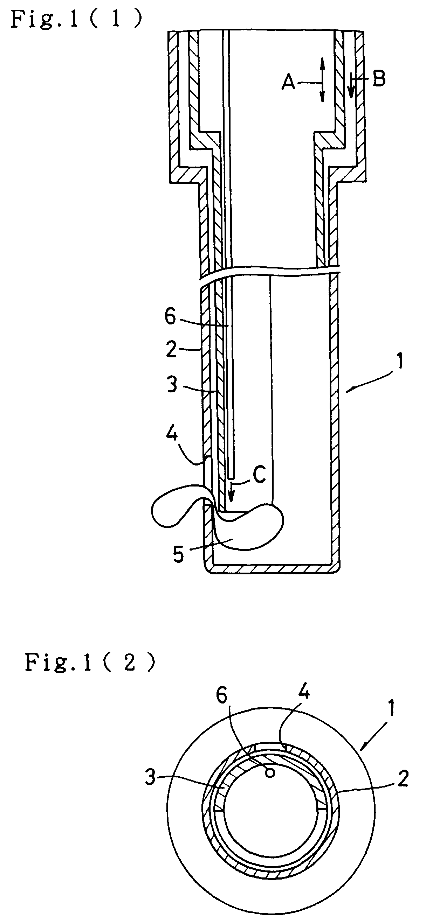

The probe-style aspirator with cutter according to a first embodiment, as illustrated in Fig. 1(1) and Fig. 1(2), comprises a hollow first probe 2 and a hollow second probe 3, which is inserted inside the first probe 2. The second probe 3 is capable of reciprocal movement with respect to the first probe 2 in the longitudinal direction of the probes 2, 3, as illustrated by arrow A in the drawings. The first probe 2 has a stepped circular cylindrical shape, one end thereof being closed and the other end being connected to a fluid supply source (omitted from drawings). The second probe 3 has a semicircular cylindrical shape at one end side thereof and a stepped circular cylindrical shape at the other end side thereof. The one end of the second probe 3 is open, whilst the other end thereof is connected to a tank (omitted from drawings). The one end of the second probe 3 has a semicircular cylindrical shape. The stepped circular cylindrical-shaped portion of each of the probes 2, 3 has a larger diameter at the other end side than one end side, thus making it possible to install an optical fiber 6, described hereinafter, inside the smaller diameter portion of the second probe 3. For example, the diameter of the larger diameter section of the first probe 2 is 0.9 mm, the diameter of the smaller diameter section thereof is 0.8 mm, the diameter of the larger diameter section of the second probe 3 is 0.6 mm, the diameter of the smaller diameter section thereof is 0.5 mm, and the diameter of the optical fiber 6 is 0.1 mm, the optical fiber 6 being installable in the second probe 3 by means of an adhesive.

The fluid supplied from the fluid supply source connected to the first probe 2 flows firstly from inbetween the first probe 2 and the second probe 3 towards the one end of the first probe 2 as illustrated by arrow B in the drawing, whereupon it flows from the one end of the second probe 3 towards the other end thereof and is finally discharged into the tank connected to the other end of the second probe 3. Thereby, a suction pressure can be created inside the first probe 2, and also the relative movement between the probes is made smooth. A physiological saline solution, for example, can be used as the fluid.

A suction inlet 4 is formed in the circular wall of the first probe 2. A portion of an object 5 to be aspirated present outside the first probe 2 can be introduced into the first probe 2 via this suction inlet 4. As described above, the portion of the object 5 can be introduced into the first probe 2 by creating a suction pressure inside the first probe 2. Alternatively, the portion of the object 5 is allowed to flow into the first probe 2 naturally.

The suction inlet 4 is opened and closed by means of relative movement of the second probe 3 with respect to the first probe 2. Fig. 1(1) shows the suction inlet 4 in a closed state. A laser cutter mechanism is provided. This mechanism separates the portion of the object 5 inside the first probe 2 from the remainder of the object 5 lying outside the first probe 2 by means of laser beam, when the suction inlet 4 is in the closed state. Specifically, an optical fiber 6, that transmits laser beam, is attached to the inner side of the second probe 3 such that it can be moved simultaneously with the second probe 3. One end face of the optical fiber 6 works as a laser beam emitting section and the other end thereof is connected to a laser beam generating source (omitted from drawings). When the suction inlet 4 is in a closed state, laser beam emitted from the end face of the optical fiber 6 as illustrated by the arrow C in the diagram is irradiated onto the portion of the object 5 inside the first probe 2. Thereby, the portion of the object 5 inside the first probe 2 is cut or disintegrated in the vicinity of the suction inlet 4 and is therefore separated from the remainder of the object 5 located outside the first probe 2. The wavelength of the laser is, for example, "1.06 µm", the intensity is approximately 1 mJ, and Nd:YAG laser device can be used as the laser beam source.

By means of the above-mentioned constitution, the portion of the object 5 inside the first probe 2 can be separated from the remainder of the object 5 located outside the first probe 2 by laser beam in the vicinity of the suction inlet 4. Therefore, the portion of the object 5 inside the first probe 2 can be aspirated reliably without causing blockages to occur. It is also possible to prevent the impacts due to the separating operation, the disintegrated object, and the laser beam from leaking outside the first probe 2, by synchronizing the laser irradiation timing of the laser cutter mechanism with the operational timing of the second probe 3 such that the suction inlet 4 is closed by means of the second probe 3 during the separation of the object 5. Safety can be thus improved in medical instrument applications, such as retinas and vitreous body surgery. By providing a sensor for detecting whether or not the suction inlet 4 is closed by the second probe 3, it is also possible to supply interlocking such that the laser beam cannot be emitted unless the suction inlet 4 is closed. Under the aforementioned suction pressure, the separated portion of the object 5 is drawn from the first probe 2 into the second probe 3 with the flow of the fluid supplied from the aforementioned fluid supply source and the fluid drawn in via the suction inlet 4, and it is finally discharged into the tank. Since the one end of the second probe 3 has a semicircular cylindrical shape, the effective diameters of both probes 2, 3 is larger, thereby preventing blockages caused by the object 5.

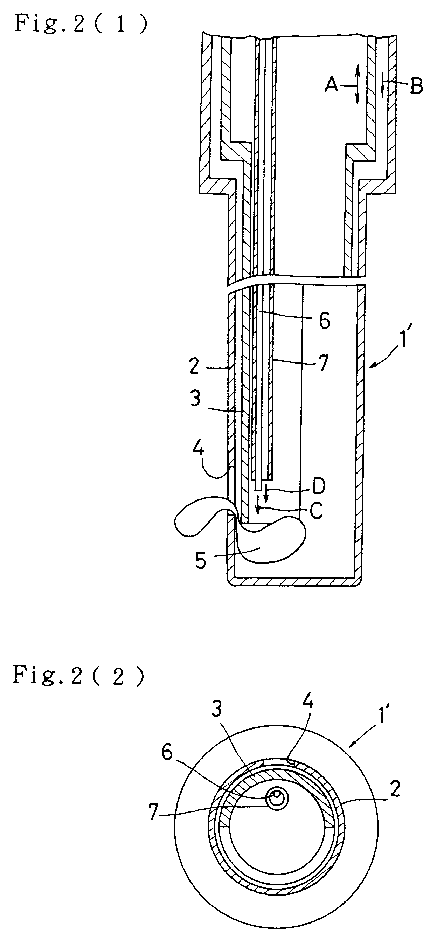

Fig. 2(1) and Fig. 2(2) shows a probe-style aspirator with cutter 1' according to a second embodiment. The point of difference with respect to the first embodiment is that a mechanism is provided for protecting the laser beam emitting section of the optical fiber 6 from contamination. Specifically, a tube 7, which conveys a fluid to the laser beam emitting section at one end of the optical fiber 6 as indicated by the arrow D in the diagram, is provided. One end of the tube 7 is positioned in the vicinity of the one end of the optical fiber 6 and the other end of the tube 7 is connected to a fluid supply source (omitted from drawings). By the flow of the fluid, contaminants contained in the separated object 5 or in the surrounding material are prevented from approaching the laser beam emitting section. Also, the contaminants, and the like, are moved away from the laser beam emitting section by the flow of the fluid. Thereby, it is possible to prevent the laser beam emitting section from becoming contaminated. In the present embodiment, since the region of one end of the optical fiber 6 is inserted into the tube 7, a flow of fluid can be formed reliably around the laser beam emitting section. By this means, it is possible to prevent the intensity of the laser beam from falling, without cleaning the laser beam emitting section. Therefore, since the laser beam can be irradiated onto the object 5 without interrupting operation for the purpose of cleaning, continuous operation becomes possible. A physiological saline solution, for example, can be used as the fluid. Apart from this, the present embodiment is the same as the first embodiment and same components are similarly labelled.

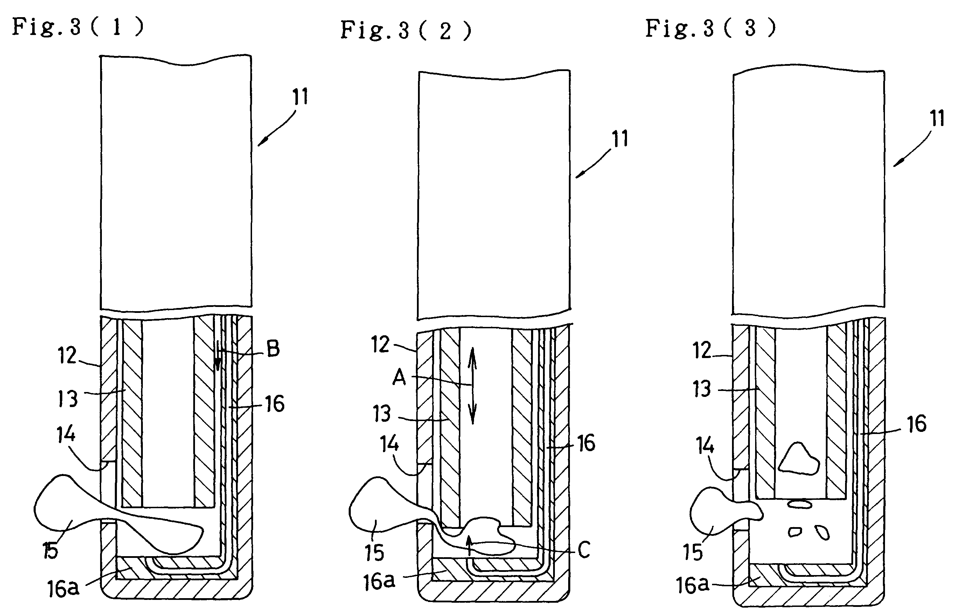

A probe-style aspirator with cutter 11 according to a third embodiment as illustrated in Fig. 3(1), Fig. 3(2) and Fig. 3(3) comprises a hollow first probe 12, and a hollow second probe 13 inserted inside the first probe 12. The second probe 13 is capable of reciprocal movement with respect to the first probe 12 in the longitudinal direction of the probes 12, 13, as illustrated by the arrow A in the drawings. The first probe 12 has a circular cylindrical shape, one end thereof being closed and the other end being connected to a fluid supply source (omitted from drawings). The second probe 13 has a circular cylindrical shape, one end face thereof being open and the other end being connected to a tank (omitted from drawings).

The fluid supplied from the fluid supply source connected to the first probe 12 flows firstly from inbetween the first probe 12 and the second probe 13 towards the one end of the first probe 12 as illustrated by arrow B in the drawing, whereupon it flows from the one end of the second probe 13 towards the other end thereof and is finally discharged into the tank connected to the other end of the second probe 13. Thereby, a suction pressure is created inside the first probe 12, and also the relative movement between the two probes 12, 13 is made smooth. A physiological saline solution, for example, can be used as the fluid.

A suction inlet 14 is formed in the circular wall of the first probe 12. A portion of an object 15 to be aspirated present outside the first probe 12 can be introduced into the first probe 12 via the suction inlet 14. In order to introduce the portion of the object 15 into the first probe 12, the suction pressure inside the first probe 12 is created as described above. Alternatively, the portion of the object 15 is allowed to flow into the first probe 12 naturally.

The suction inlet 14 is opened and closed by relative movement of the second probe 13 with respect to the first probe 12. Fig. 3(1) shows the suction inlet 14 in an open state, and Fig. 3(2) shows it in a closed state. When the suction inlet 14 is in an open state, the portion of the object 15 is introduced into the first probe 12. Thereupon, by relative movement of the second probe 13 with respect to the first probe 12 such that the suction inlet 14 is closed, a shearing force is applied to cut the portion of the object 15 inside the first probe from the remainder of the object 15 located outside the first probe 12. A mechanism for disintegrating the portion of the object 15 inside the first probe 12 is provided in the vicinity of the suction inlet 14, when the suction inlet 14 is in a closed state, such that there is no blocking of the probes 12, 13. Specifically, an optical fiber 16, which transmits laser beam, is attached to the first probe 12 between the first probe 12 and the second probe 13. One end of the optical fiber 16 works as a laser beam emitting section positioned such that it opposes one end of the second probe 13. The other end of the optical fiber 16 is connected to a laser beam generating source (omitted from drawing). The optical fiber 16 is covered by a protective cover 16a. Laser beam is emitted from the one end of the optical fiber 16 as illustrated by arrow C in the diagram. As shown in Fig. 3(3), the portion of the object 15 inside the first probe 12 is disintegrated in the vicinity of the suction inlet 14 by irradiation of the laser beam. Under the aforementioned suction pressure, this disintegrated portion of the object 15 is drawn from the first probe 12 into the second probe 13 with the flow of the fluid supplied by the aforementioned fluid supply source and the fluid drawn in via the suction inlet 14, and finally it is discharged into the tank.

By means of the above-mentioned constitution, even if the portion of the object 15 located inside the first probe 12 cannot be separated from the remainder of the object 15 located outside the first probe 12 by the cutting, it is disintegrated in the vicinity of the suction inlet 14 by the laser beam. Thereby, the portion of the object 15 inside the first probe 12 can be aspirated reliably without causing blockages. In the present embodiment, it is also possible to prevent the impacts due to the disintegrating operation, the disintegrated object, and the laser beam from leaking outside the first probe 12, by synchronizing the disintegrating timing of the disintegrating mechanism with the operational timing of the second probe 13 such that the suction inlet 14 is closed during the disintegrating of the object 5. Thereby, safety can be improved in medical equipment applications, for instance, when the device is used in retinas and vitreous body surgery, and the like.

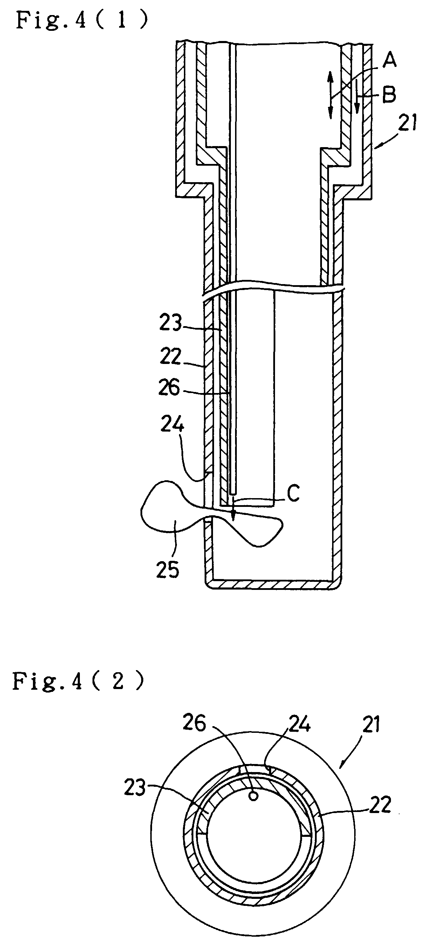

The probe-style aspirator with cutter 21 according to a fourth embodiment, as illustrated in Fig. 4(1) and Fig. 4(2), is used in retinas and vitreous body surgery in the field of ophthalmology, and it comprises a hollow first probe 22 and a hollow second probe 23 which is inserted into the first probe 22. The second probe 23 is capable of reciprocal movement with respect to the first probe 22, in the longitudinal direction of the probes 22, 23, as indicated by arrow A in the diagram. The first probe 22 has a stepped circular cylindrical shape, one end thereof being closed and the other end being connected to a fluid supply source (omitted from drawings). The second probe 23 has a semicircular shape at one end side thereof and a stepped circular cylindrical shape at the other end side thereof. The one end of the second probe 23 is open and the other end is connected to a tank (omitted from drawings). The one ends of both probes 22, 23 are inserted into the eyeball, and the other ends thereof are located outside the eyeball. The one end side of the second probe 23 has a semicircular cylindrical shape, and each of the probes 22, 23 has a stepped circular cylindrical shape so that its large diameter portion is located outside the eyeball, it is thus possible to attach an optical fiber 26, described hereinafter, to the inner side of the small diameter portion of the second probe 22.

As shown by arrow B in the diagram, fluid supplied by the fluid supply source connected to the first probe 22 flows from inbetween the first probe 22 and the second probe 23 towards the one of the first probe 22, whereupon it flows from the one end of the second probe 23 towards the other end thereof, and is finally discharged into the tank connected to the other end of the second probe 23. Thereby, a suction pressure is created inside the first probe 22, and also the relative movement between the two probes is made smooth. A physiological saline solution, for example, can be used as the fluid.

A suction inlet 24 is formed in the circular wall of the first probe 22. A portion of a vitreous body 25, which is the object to be aspirated, present outside the first probe 22 can be introduced into the first probe 22 via this suction inlet 24. As described above, a portion of the vitreous body 25 is introduced into the first probe 22 by creating the suction pressure inside the first probe 22. Alternatively, a portion of the vitreous body 25 is allowed to flow inside the first probe 22 naturally.

The suction inlet 24 is opened and closed by relative movement of the second probe 23 with respect to the first probe 22. Fig. 4(1) shows the suction inlet 24 in an open state. A portion of the vitreous body 25 is introduced into the first probe 22 whilst the suction inlet 24 is in an open state, and then a shearing force is applied to the portion of the vitreous body 25 inside the first probe 22 in order to cut it from the remainder of the vitreous body 25 outside the first probe 22, by causing the second probe 23 to move relative to the first probe 22 such that the suction inlet 24 closes. A disintegrating mechanism is provided which disintegrates the portion of the vitreous body 25 inside the first probe 22 in the vicinity of the suction inlet 24, when the suction inlet 24 is in a closed state, such that the portion of the vitreous body 25 does not block up the probes 22, 23. Specifically, an optical fiber 26, which transmits laser beam, is installed on the inner side of the second probe 23 such that it can be moved simultaneously with the second probe 23. One end of the optical fiber 26 works as a laser beam emitting section and the other end thereof is connected to a laser beam generating source (omitted from diagram). Laser beam is emitted from the one end of the optical fiber 26 as illustrated by the arrow C in the diagram. The portion of the vitreous body 25 inside the first probe 22 is disintegrated by irradiation of laser beam. Under the aforementioned suction pressure, the disintegrated portion of the vitreous body 25 is drawn from the first probe 22 into the second probe 23 with the flow of fluid supplied from the aforementioned fluid supply source and the fluid drawn in via the suction inlet 24, and it is finally discharged into the tank.

By means of the foregoing constitution, even if the portion of the vitreous body 25 inside the first probe 22 cannot be cut from the remainder of the vitreous body 25 located outside the first probe 22 by the shearing force, it can be aspirated reliably without causing blockages, by disintegrating it in the vicinity of the suction inlet 24 by means of laser beam. Thereby, a vitreous body can be aspirated efficiently, without the suction inlet 24 being made unnecessarily small. Moreover, since the surgeon is not required to pay attention with respect to blocking of the probes, the surgery time is shortened and hence the load on the patient can be reduced. Furthermore, by synchronizing the disintegrating timing of the disintegrating mechanism with the operational timing of the second probe 23, such that the suction inlet 24 is closed during the disintegrating, it is possible to prevent the impacts due to the disintegrating operation, the disintegrated object, and the laser beam from leaking outside the first probe 22. Thereby, safety can be raised in retinas and vitreous body surgery applications. Moreover, by giving one end side of the second probe 23 a semicircular shape, the effective diameter of the probes 22, 33 is increased, and blockages caused by the vitreous body 25 can be prevented.

The present invention is not limited to the foregoing embodiments. For example, the probe-style aspirator with cutter according to the present invention is not limited to separating and aspirating vitreous matter in the field of ophthalmology, but can also be used in other applications. Furthermore, the relative movement of the second probe with respect to the first probe is not limited in particular, provided that the effect of the present invention can be obtained; for example, it can be a rotational movement or it can combine a rotational movement and a linear movement. The operation of the first probe 2 can be controlled manually by a user or it can be driven by a driving mechanism. The method for aspirating the object to be aspirated is not particularly limited, and an optimum device and method should be adopted in accordance with the objective. In the foregoing embodiments, the suction inlet is formed on the circular wall of the first probe, but it can be formed such that it is an opening at the tip of the first probe. In this case, the suction inlet can be opened and closed by rotating the second probe with respect to the first probe, and a section for applying a shearing force to the object to be aspirated can be provided in the tip portion of the second probe. Laser beam for irradiation onto the object to be aspirated can be emitted through a lens system provided in the tip portion of the optical fiber in order that it is irradiated efficiently. The lens system can be composed by rod lenses or by processing the end face of the optical fiber. The diameter and material of the optical fiber is not limited, and the optimum known material for the application is chosen. The wavelength of the laser beam is chosen in accordance with the object to be aspirated. The type of laser is selected such that it suits the application. Moreover, the method for controlling the laser generating device is not limited in particular, and it can be controlled by a continuous wave (CW) or pulse wave. The positioning of the laser cutter mechanism according to the first and second embodiments is not limited in particular, and also the direction of laser beam emission is not limited in particular, provided that the portion of the object 5 inside the first probe 2 can be separated from the remainder of the object 5 outside the first probe 2. The positioning of the disintegrating mechanism in the third and fourth embodiments is not limited in particular, and also the direction of laser beam emission is not limited in particular, provided that a portion of the object to be aspirated can be disintegrated prior to aspiration in the second probe. In the third and fourth embodiments, it is not necessarily to synchronize the disintegrating timing of the disintegrating mechanism with the operational timing of the second probe, and the user can synchronize these as necessary. Furthermore, the optical fiber in the third and fourth embodiments can be provided with a protective mechanism preventing contamination of the laser beam emitting section, similarly to the second embodiment. Moreover, the disintegrating mechanism in the third and fourth embodiments is not limited to using a laser, for example, a mechanism, which disintegrates the object to be aspirated mechanically, can be adopted.

## Claims

1. A probe-style aspirator with cutter, comprising:
a hollow first probe (2);
a second probe (3) inserted into said first probe (2) such that it is capable of moving relative to the first probe (2);
a suction inlet (4) formed in said first probe (2), in order that a portion of an object (5) to be aspirated present outside the first probe (2) can be introduced into the first probe (2) via the suction inlet (4); the suction inlet (4) being opened and closed by relative movement of the second probe (3) with respect to the first probe (2); and
a laser cutter mechanism which separates the portion of the object (5) inside the first probe (2) from the remainder of the object (5) outside the first probe (2) by means of laser beam, when the suction inlet (4) is in a closed state.

2. A probe-style aspirator with cutter, comprising:
a hollow first probe (12, 22);
a second probe (13, 23) inserted into said first probe (12, 22) such that it is capable of moving relative to the first probe (12, 22);
a suction inlet (14, 24) formed in said first probe (12, 22), in order that a portion of an object (15, 25) to be aspirated present outside the first probe (12, 22) can be introduced into the first probe (12, 22) via the suction inlet (14, 24), and a shearing force can be applied to the portion of the object (15, 25) inside the first probe (12, 22) by relative movement of the second probe with respect to the first probe (12, 22) to separate it from the remainder of the object (15, 25) outside the first probe (12, 22); and
a mechanism for disintegrating the portion of the object (15, 25) inside the first probe (12, 22) in the vicinity of said suction inlet (14, 24), in order that a blockage inside the probes is not caused.

3. The probe-style aspirator with cutter according to claim 2, wherein said suction inlet (14, 24) is opened and closed by relative movement of the second probe (13, 23) with respect to the first probe (12, 22); and said mechanism disintegrates the portion of the object (15, 25) inside the first probe (12, 22) by laser beam, when the suction inlet is in a closed state.

4. The probe-style aspirator with cutter according to any of claims 1 to 3, comprising an optical fiber (6, 26) for emitting laser beam, wherein the laser beam emitting section of said optical fiber (6, 26) is prevented from contamination by a fluid conveyed thereto.
